# EUROPEAN PATENT APPLICATION

(11) **EP 3 854 440 A1**
(43) Date of publication of application: **28.07.2021**
(21) Application number: 19205756.0
(22) Date of filing: 28.10.2019
(51) Int. Cl.: A61M 27/00, A61B 10/00, B01D 61/18, A61F 2/01

(54) **AN IMPLANTABLE DEVICE FOR THE INTRACRANIAL SPACE OF A LIVING BEING FOR ATTRACTING AND HOLDING PATHOGENIC PARTICLES**

(71) Applicant: Meta, Abdulla, 6003 Lucerne (CH)
(72) Inventor: Meta, Abdulla, 6003 Lucerne (CH)
(74) Representative: Grosfillier, Philippe

(57) **Abstract**

An implant device for placement into a brain cavity of a living being between the meninges and the cerebrum to be in contact with a cerebro-spinal fluid, the implant device including a magnet structure having a biocompatible coating, and a guiding structure made of a biocompatible material or coated with a biocompatible material, an outer surface of the guiding structure configured to be in contact with the cerebro-spinal fluid and forming a collection area, wherein the guiding structure includes at least one channel having an opening at an outer surface of the guiding structure that is configured to face the collection area.

## Description

### FIELD OF THE INVENTION

The present invention is directed to the field of brain implants and methods implanting a brain implant, and methods associated thereto for capturing pathogenic particles, for example for the treatment of paratonia and brain tumors.

### BACKGROUND

In the field of the treatment of the human body for brain tumors of a living being, mostly intrusive radiation therapies are used, that can lead to lasting damage to the brain. Specifically, radiation therapies can severely affect any tissue or volumes of the brain that lie in the path of the radiation beam, usually volumes that are adjacent to a location of the tumor. Other types of brain surgery are also performed for tumor removal, for example by craniotomy to mechanically remove the entire tumor, but these operations can also lead to lasting damage of the brain, as sometimes some parts of the tumor need to remain to preserve specific functions of the brain, depending on a location of the tumor. Chemotherapy is another option, that can involve the injection of drugs into the cerebrospinal fluid of the brain, but have shown to have many adversarial side effects that can affect the entire human body.

With respect to the causation of different types of brain tumors, it has been shown that fine particles, and other type of particulate matter (PM) that are absorbed or otherwise brought into a body of a living being, for example a human, have strongly contributed to the formation of brain tumors. For example, it has been shown that different heavy metals can lead to epigenetic alterations and brain tumors, for example DNA damage, lipid peroxidation, and alteration of proteins. For example, presence of iron exposure, chromium, and cadmium has led to augmented risks of cerebral tumors. See Caffo et al. "Heavy Metals and Epigenetic Alterations in Brain Tumors." Current Genomics Vol. 15, No. 6, 2014, pp. 457-463. Moreover, the exposure to lead, cadmium, mercury, and arsenic has shown to severe adverse health effects on humans, and it has been shown that lead exposure due to high gastrointestinal uptake, for example from the drinking of polluted water or water from lead pipes, can enter the body and pass through the permeable blood-brain barrier, reaching the cerebrospinal fluid. See Lars Järup, "Hazards of Heavy Metal Contamination," British Medical Bulletin Vol. 68, No. 1, 2003, pp. 167-182

In addition, it has been shown that different metal and silicate particles are ingested by human beings by the smoking of cigarettes, but also from vaping and other types of E-cigarettes. See Williams et al., "Metal and Silicate Particles Including Nanoparticles are Present in Electronic Cigarette Cartomizer Fluid and Aerosol," PloS one Vol. 8, No. 3, 2013, e57987. Specifically, it has shown that the aerosol of electronic cigarettes that is ingested by users contain metals that are derived from various components of the liquid, and the aerosol contained particles >1 µm comprised of tin, silver, iron, nickel, aluminum, and silicate and nanoparticles (<100 nm) of tin, chromium and nickel. SEE Bleise et al., "Properties, Use and Health Effects of Depleted Uranium (DU): a General Overview," Journal of Environmental Radioactivity, Vol. 64, No. 2-3, 2003, pp. 93-112. These particles in turn can end up in the CSF of a living being.

Similarly, living beings that are suffering from paratonia, usually suffer of a frontal lobe disfunction of the brain. The results are severe movement dysfunction in late stage dementia. It has been shown that advanced glycation end-product ("AGE") have contributed to paratonia and the decline in motor function. Drenth et al., "Advanced Glycation End-Products are Associated with the Presence and Severity of Paratonia in Early Stage Alzheimer Disease," Motor Function, Paratonia and Glycation Cross-linked in Older People: p. 61, 2017. The AGE are a result of pathologic conditions, such as oxidative stress due to hyperglycemia in patients with diabetes, and can be at least partially removed by cellular proteolysis of AGE that leads to the breakdown of proteins. However, it has been shown that this type of AGE removal only shows limited success. Gugliucci et al., "Paraoxonase-1 Concentrations in end-stage Renal Disease Patients Increase after Hemodialysis: Correlation with Low Molecular AGE Adduct Clearance," Clinica Chimica Acta, Vol. 377, No. 1-2, 2007, pp. 213-220.

Therefore, in light of the above-discussed deficiencies of the treatment of brain tumors and paratonia, and the treatment of certain causes of these diseases related to fine particles that are present in the cerebro-spinal fluid, there is a strong need for substantially improved solutions for containing or removing metal particles from the body of a living being, for example by providing an implant device and methods for treating patients or living beings for at least partially capturing and isolating particles.

### SUMMARY

According to one aspect of the present invention, an implant or implantable device for placement into a brain cavity of a living being between the meninges and the cerebrum to be in contact with a cerebro-spinal fluid is provided. Preferably, the implant device includes a magnet structure having a biocompatible coating, and a guiding structure made of a biocompatible material or coated with a biocompatible material, an outer surface of the guiding structure configured to be in contact with the cerebro-spinal fluid and forming a collection area, wherein the guiding structure includes at least one channel having an opening at an outer surface of the guiding structure that is configured to face the collection area.

According to another aspect of the present invention, a cerebro-spinal brain fluid treatment system is provided. Preferably, the system includes a magnet device to be placed in close proximity of a skull of a living being, and an implantable guiding structure made of a biocompatible material or coated with a biocompatible material, configured to be in contact with the cerebro-spinal fluid and forming a collection area. Moreover, preferably the implant structure includes a base portion and, a protruding body element, and at least one channel, each one of the plurality of channels having an opening at an outer surface of the implantable guiding structure that is configured to face the collection area.

The above and other objects, features and advantages of the present invention and the manner of realizing them will become more apparent, and the invention itself will best be understood from a study of the following description with reference to the attached drawings showing some preferred embodiments of the invention.

### BRIEF DESCRIPTION OF THE SEVERAL VIEWS OF THE FIGURES

The accompanying drawings, which are incorporated herein and constitute part of this specification, illustrate the presently preferred embodiments of the invention, and together with the general description given above and the detailed description given below, serve to explain features of the invention.
FIGs. 1A to IE show different views according to a first embodiment of an exemplary implant device 100, with FIG. 1A showing a cross-sectional side view thereof, along a line B-B that is shown in FIG. 1C, FIG. 1B showing a side view thereof, FIG. 1C shows a bottom view thereof, FIG. ID shows a top view thereof, and FIG. IE showing a top, cross-sectional view of the implant device 100;
FIG. 2 shows a side view of an exemplary implant device 100 having protruding columns 12 that are arranged in brain fissures 64 that are adjacent to a tumor-affected brain gyrus 63 having a tumor 50;
FIG. 3 shows an second embodiment of an exemplary implant device 200, where an upper section of guiding structure 210 serves as a holding and attachment mechanism for the magnet structure 220;
FIG. 4 shows an exemplary cross-sectional view of a brain and skull of a living being, with implant device 100, 200 placed into the subarachnoid space as the brain cavity, showing a tumor 50 inside a brain gyrus, with columns 12, 212 of guiding structure 10 protruding into tumor-neighboring brain fissures 64;
FIGs. 5A-5C show different views of an another embodiment of implant device 300, where the legs 312 of guiding structure 310 are formed to be longitudinal and bent or curved tabs, and magnets 322, 324 are arranged to be at different angles towards each other, FIG. 5A showing a cross-sectional view, FIG. 5B shows a perspective view, and FIG. 5C shows a cross-sectional view of a brain with the placement of implant device 300;
FIG. 6 shows an exemplary system 400 that uses an implant device 410 that is made of guiding structure with a collection cavity, and an external magnet 420, for example an electromagnet, for providing the magnetic field to guiding structure 410 of implant system 400; and
FIG. 7 shows an exemplary cross-sectional view of another embodiment of implant device 500 having a single central cavity or opening 514 that leads to a collection chamber 526 that is arranged between a lower surface of a single magnet 520.

Herein, identical reference numerals are used, where possible, to designate identical elements that are common to the figures. Also, the images are simplified for illustration purposes and may not be depicted to scale.

### DESCRIPTION OF THE SEVERAL EMBODIMENTS

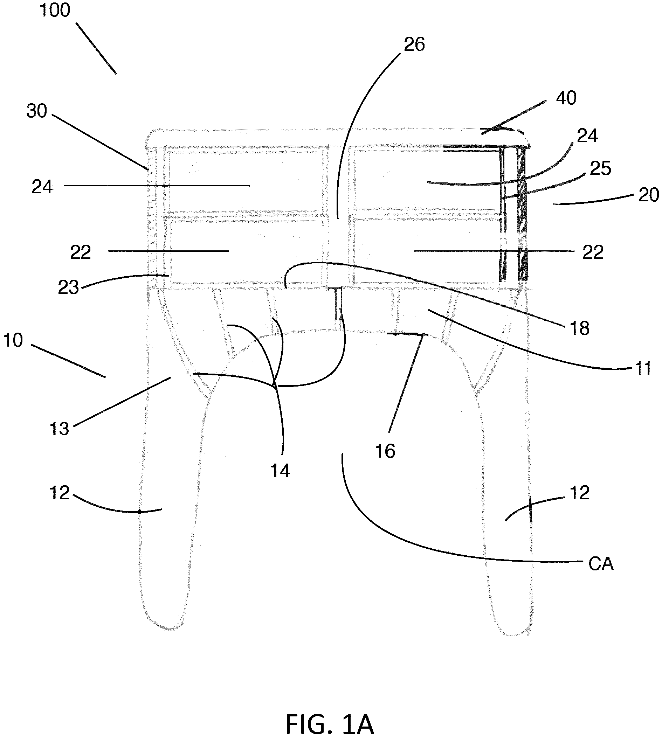

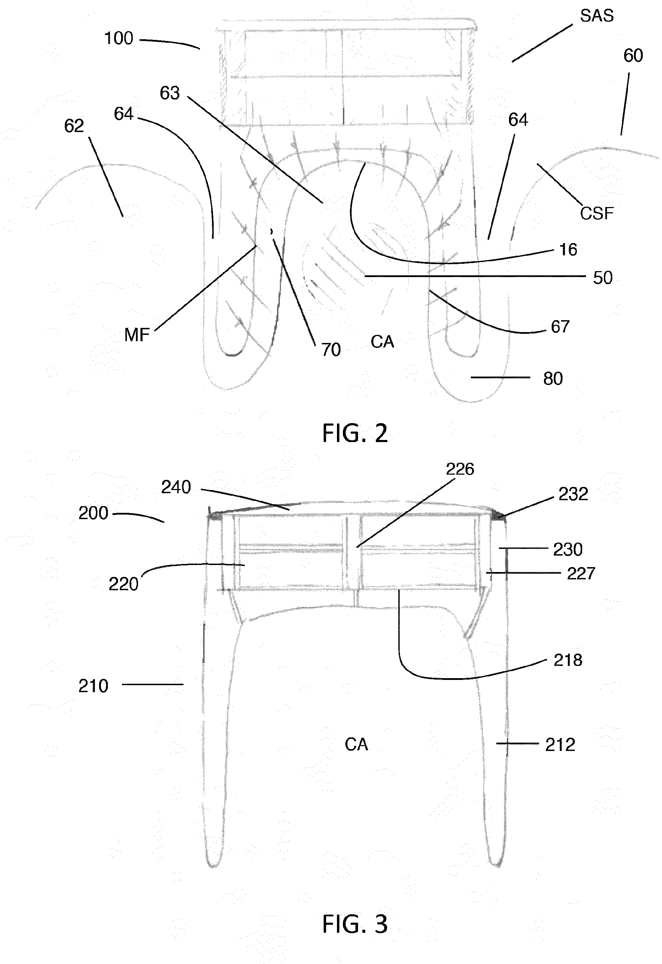

According to an aspect of the present invention, an exemplary implant device 100 is provided, as shown in FIGs. 1A to IE, with cross-sectional views provided by FIGs. 1A and IE. Implant device 100 serves to attract and collect particles 70 in the cerebro-spinal fluid (CSF) and includes a guiding structure or element 10 with protruding columns 12 that can be implanted to a head of a living being, specifically a human being, with its outer surface being in contact with the CSF as exemplarily shown in FIG. 2, located between cerebral cortexes (brain or cerebrum 60), between the arachnoid mater and the pia mater, preferably in the subarachnoid space SAS. Between protruding columns 12, a collection area CA is formed, being an area where particles 70 can be located. Upon implantation, each one of protruding columns 12 of guiding structure 10 of implant device 100 are configured to be arranged inside two or more longitudinal fissures or sulci 64 that are adjacent to a brain gyrus 63, brain gyrus 63 potentially being affected by a tumor 50. In the variant shown in FIGs. 1A-1E, implant device 100 has four (4) protruding columns 12, but it is possible that there are more or less than four (4), with the minimum of two (2) columns 12 for placement into brain fissures 64, to form an accommodating space for brain gyrus 63. The exemplary implant device 100 shown can be said to have a four-legged stool-like or table-like shape, with columns 12 arranged at corners of the partially square-shaped base portion or plate 11 of guiding structure 10. However, base plate 11 can have different shapes, for example round, elliptic or a polygon shape, and a location of protruding columns 12 need not be at corners, but can be somewhat in the interior of base plate 11. Also, preferably, corners of plate 11 are rounded.

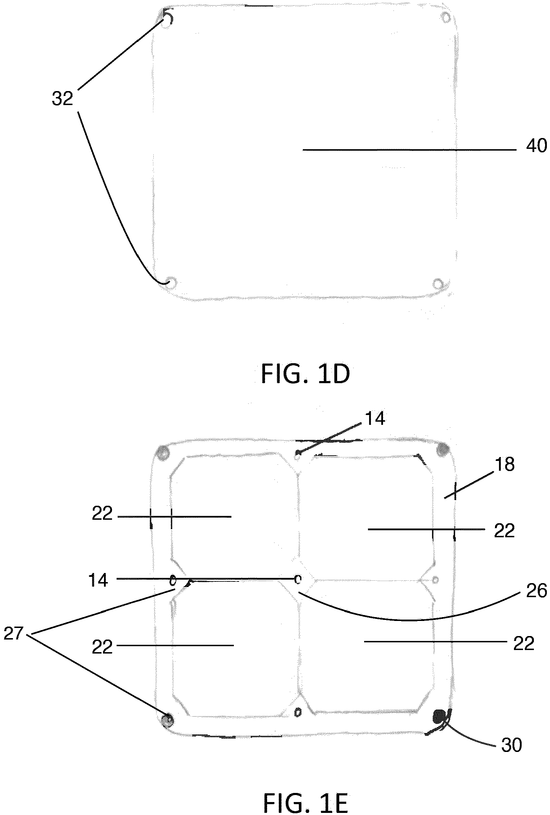

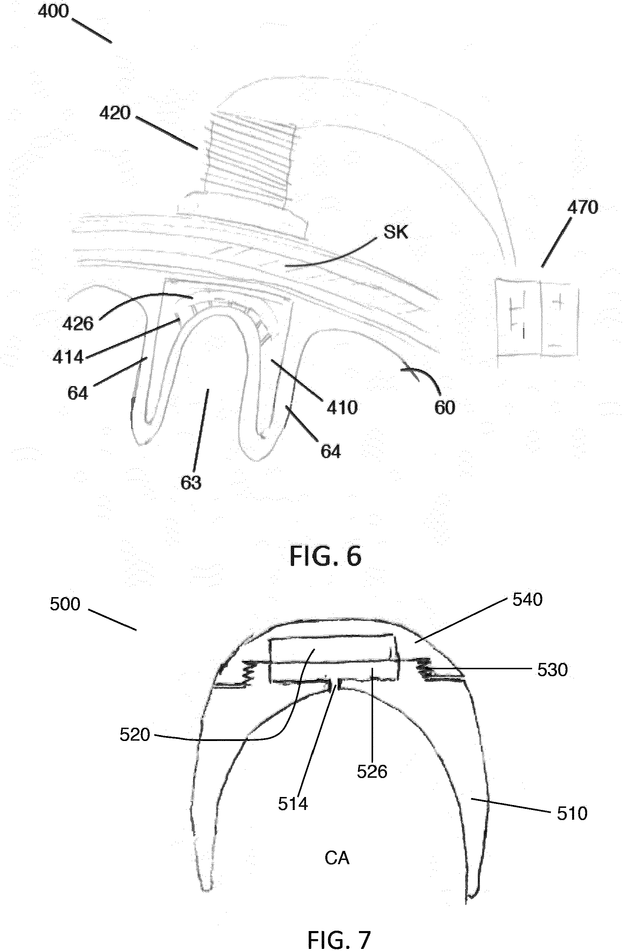

Implant device 100 includes a magnet structure 20 and a guiding structure 10, and attachment means 30, 40 to attach the magnet structure 20 to the guiding structure 10. Magnet structure 20 includes a plurality of magnets 22, 24, the magnets arranged in a stack, the stack including a lower layer of magnets 22 having a lower surface that is in contact with an upper surface 18 of guiding structure 10, and having an upper layer of magnets 24 that are held to be in contact with corresponding magnets 22 of the lower layer of magnets 22. However, it is also possible that the lower surface of magnets 22 are not in direct contact with guiding structure 10, and there is a gap inbetween magnets 22 and upper surface 18 of guiding structure 10, to form a collection chamber (FIGs. 6 and 7, 426, 526) between lower surface of magnets 22 and upper surface 18 of guiding structure 10. It is also possible that there is only one magnet instead of a stack of magnets 22, 24. Magnets 22, 24 are preferably permanent magnets, for example ones that are made of neodymium, generating a magnetic field having a magnetic flux density in the range between 0.1 to 1.4 Tesla. Attachment means 30, 40 includes a lid 40 for holding the stack of magnets or magnet structure 20 together, so that they are tightly interconnected to each other, and also tightly connected to upper surface 18 of the guiding structure 10, and fixation columns 30 that are exemplarily shown to be arranged at the four (4) corners of the exemplary square-shaped cross-section of the implant device 100, see FIG. 1D, IE. Columns 30 can be attached to base plate 11 and lid 40 by different means, for example but not limited to by a threading, a nut, a snap-in mechanism, locking pins, by the use of a biocompatible adhesive, or by a press-fit mechanism, or a combination thereof. Columns 30 can also be made by different means, for example but not limited to brackets, threaded bolts, pins, holders, screws.

The use of the terms "upper," "lower," "top," "bottom," "downwards," and "upwards," refer to the representation of the device 100 as shown in the figures, and the potential implantation of device 100 with the guiding structure 10 facing downwards towards a brain mass 60 and lid 40 of attachment means 30, 40 facing upwards towards the skull bone SK of the living being, as shown in FIG. 2. However, depending on a place of implantation of the living being, for example at sides of the brain, or depending on an orientation of a living being itself, these geometric references may be different.

All the elements 10, 20, 30, and 40 are made or are at least coated by a biocompatible coating layer. For example, all magnets 22, 24 from the magnet structure 20 can be coated by a thin coating layer, for example one that is thinner than 0.2 mm, to provide for biocompatibility, and mechanical and chemical stability. This can also be a two layer structure, having an inner layer for mechanical and chemical stability for example to prevent corrosion such as a nickel plating, and an outer layer for biocompatibility. For example, each individual magnet 22, 24 of the magnet structure 20 can be coated with a coating layer 25 of biocompatible material, for example by a titania film. See for example Kern et al., "Electrolytic Deposition of Titania Films as Interference Coatings on Biomedical Implants: Microstructure, Chemistry and Nano-Mechanical Properties," Thin Solid Films, Vol. 494, No. 1-2, 2006, pp. 279-286. The coating layer 25 of the magnets 22, 24 can also be made in different colors, for example red, blue, green, and yellow. The colors can serve for identification purposes of the magnets 22, 24 and orientation of device 100, but also for additional biocompatibility purposes.

Moreover, magnets 22, 24 and the stack of magnets or magnet structure can be arranged to have some open passages arranged between them. For example, in the variant shown in FIG. 1A, there is a central passageway or chamber 26 that forms an open space between side walls of adjacent magnets 22, 24 at a center, adjacent to four (4) neighboring magnets, also shown from a top view in the cross-section of FIG. IE. This passageway 26 can be created by having the otherwise square-shaped magnets 22, 24 with rounded, beveled or otherwise cut edges, or by using cylindrical magnets. In addition, additional lateral passageways 27 can be present at the sides of the magnet structure 20, as visualized in FIG. IE. The amount of magnets 22, 24 chosen to be four (4) is only exemplary, and more magnets could be used for each stack, preferably each layer of the stack having the same amount of magnets. It is also possible that magnets 22, 24 have holes through the magnets 22, 24 themselves to provide for passageways 26, 27, for example additional ones.

With respect to guiding structure 10, this element has at least one protruding column 12, in the variant shown four (4) columns arranged at each corner of the square-shaped cross-section of the implant device 100, the columns 12 protruding downwardly away from base plate 11 of guiding structure 10. Columns 12 are shown to protrude at a perpendicular angle from base plate 11, but it is also possible that columns 12 are arranged obliquely to base plate 11. The guiding structure 10 includes a plurality of channels or cavities 14 that provide for a fluidic connection between lower surface 16 of guiding structure 10 and the upper surface 18, through base plate 11, portions of upper surface 18 being in direct contact or close proximity with lower surface of magnets 22. In a variant, channels 14 are not fully traversing, but have openings only on lower surface 16 of guiding structure 10. In another variant, ends of channels 14 inside base plate end in one or more collection chambers 426, see FIG. 6. In a variant, an entrance orifice of channels 14 can have tapered or funneled shape to better capture flowing particles 70. In another variant, one or more channels 14 are arranged at the apex of the concave or curved lower surface 16, to lead to central chamber or passageway 26, for example as shown with the central channel in FIG. 3 or FIG. 7. Preferably, lower surface 16 that lies within an area delineated by columns 12 is concave for particle capturing and collection purposes.

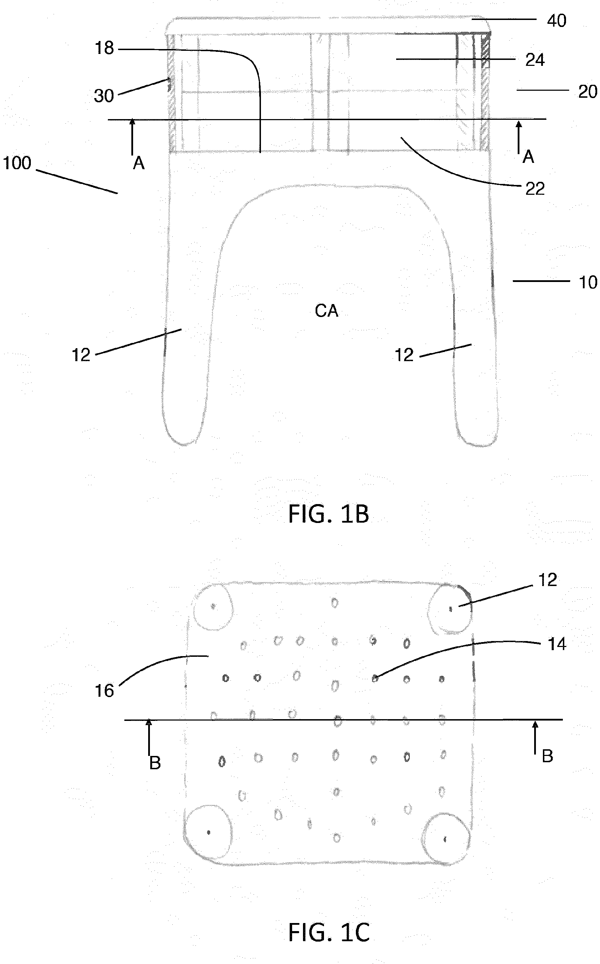

Some of the exit orifices of channels 14 can be aligned with a location of passageways 26, 27. As shown in FIG. 1C, the exemplary embodiment has thirty-six (36) channels 14, with five (5) of these channels having an exit orifice that leads to central passageway 26 and side passageway 27. Instead of channels 14 in base plate 11, it is also possible to use a base plate 11 having a porous body between surface 16 and 18, to provide for a variety of fluidic channels between surface 16 and surface 18, for example a porous body part at the surface area of the inlet orifices of channels 14, as shown in FIG. 1C, or an array of microchannels. Moreover, it is also possible that an entrance orifice, exit orifice, or a middle section of the fluidic duct formed by channel 14 be equipped with a membrane or valve configured to let particles 70 pass to approach the magnet structure, but will not allow particles to exit membrane or valve.

Guiding structure 10 with base plate or center body portion 11 and columns 12 can be made of a biocompatible material or coated with a biocompatible material. For example, guiding structure 10 can be made of a ferromagnetic core, and can be coated by a biocompatible precious metal that does not have ferromagnetic properties, for example palladium, or by other types of biocompatible precious metals, and biocompatible alloys thereof. See Brian Woodward, "Palladium in Temporary and Permanently Implantable Medical Devices," Platinum Metals Review, Vol. 56, No. 3, 2012, pp. 213-217, and Johnson et al., "Biocompatibility of precious metals for medical applications," In Precious Metals for Biomedical Applications, pp. 37-55. Woodhead Publishing, 2014, these references incorporated by reference in their entirety. For example, ferromagnetic core 13 can be made of iron, cobalt, nickel and most of their alloys, and some compounds of rare earth metals that have ferromagnetic properties, or other types of ferromagnetic materials.

After attracting of particles 70 located within CSF in collection area CA by magnetic structure 20, particles can arrive at entrance or input orifices of the channels 14, and channels 14 can serve as ducts, conduits, or lumens to guide particles 70 towards magnet structure 20 by magnetic attraction. Therefore, inner walls of channels 14 form fluidic pathways for the particles 70 in CSF, and need to be coated or otherwise made biocompatible for the body implant environment and should provide a barrier or distance layer to the ferromagnetic core 13, for example by a biocompatible material that is not ferromagnetic. This allows to avoid adherence of particles 70 to any part of the surface that is in contact with CSF, and thereby avoid interference with a particle flow within CSF inside channels 14. In some variant, center body portion or base plate 11 is arranged to have its upper surface 18 being in contact with lower surface of the lower stack of magnets 22. Preferably, in case a ferromagnetic core 13 is used for structure 10, a distance between contact surface of base plate 11 with lower surface of magnets 22 should be made small, so that the coating thickness needs to be very thin, for both magnets 22 and base plate 11, to provide for a good magnetic connection and flux between base plate 11 and magnets 22. However, in a variant, there is a direct connection by these two surfaces, and the magnets 22 and upper surface of base plate 11 are not coated at these contact areas, to maximize contact for improved magnetic flux.

Moreover, the outer lower surface 16 of guiding element 10 and columns 12 forms a smooth, curved surface, and does not have any sharp edges or corners. For example, a cross-sectional shape of columns 12 can be a circle, ellipse, or other rounded surface having no sharp edges, and base plate 11 has a smooth concave surface with the entry orifices of channels 14 having rounded edges. This feature allows for implantation of device 100 to a brain gyrus 63 with adjacent brain sulci 64, preferably brain fissures 64 that will accommodate the columns 12, without having any sharp pressure points at the outer surface that could cause any injury. Also, ends of columns 12 are designed to be round, for example to form a semispherical end surface, or other type of rounded surface. Similarly, lid 40 is also provided with rounded or otherwise smoothed edges and corners. It is also possible that side walls of magnets 22, 24 that face the external environment, for example the CSF when implanted, are encapsulated with a biocompatible filler or paste, to be used as a mechanical protective material. Exemplarily, this can be bone-tissue engineering material. See for example Burg et al., "Biomaterial Developments for Bone Tissue Engineering," Biomaterials Vol. 21, No. 23, 2000, pp. 2347-2359. Moreover, concave surface 16 of base plate 11 that faces brain gyrus 63 can be made to have a curvature radius that substantially matches a radius of the outer surface of the corresponding brain gyrus 63, for fitted accommodation to the implant site or location, for example a location of brain gyrus 63 affected by tumor 50. The concavity of surface 16 also serves as a funnel or funneling effect to make sure particles 70 attracted by magnetic field MF inside the collection area CA, and will be directed to one or more channels 14 and towards surfaces of magnets 22, 24, to be accumulated therein, and will not attach or otherwise be guided towards outer surfaces of guiding element 10.

With respect to the dimensions of implant, the dimensions shown in the figures are merely chosen for visualization purposes, and need not correspond to the preferred dimensions of a possible embodiment. For example, the dimensions can be chosen specific to the implant site or location, taking into account a thickness or depth of subarachnoid space SAS at a location of tumor 50, a width of the affected or corresponding gyrus 63 by tumor 50, a depth and width of fissures or sulci 64, a radius or shaped of curvature of an outer surface of brain gyrus 63. For example, the upper section of implant device 100 that includes lid 40, magnet structure 20 and base plate 11 can be made to have a small height or low profile, preferably less than 5 mm, so that it can fit into the subarachnoid space SAS, without adding any substantial pressure to brain 60 that could cause discomfort to the living being having implant device 100. This could lead to individual magnets having a thickness or depth of less than 2 mm. With respect to the width of the entire implant device 100, preferably the width is such that a distance between columns 12 covers a width formed by two fissures or sulci 64 that are adjacent to a brain gyrus 63, for example preferably less than 3 cm. With respect to a width of columns 12, the width is chosen to allow for easy protrusion into brain fissures 64, preferably a width of less than 3mm, and tapered towards the bottom. With respect to a depth of the protruding part of guiding element 10, i.e. columns 12, preferably the depth is less than 3cm. Columns 12 need not be designed to fully protrude into the sulci or fissures 63, but to cover at least 50% of its depth. With respect to a width or diameter of a lumen that is formed by channels 14, an exemplary diameter can have microscopic dimensions, for example smaller than 0.5 mm, more preferably smaller than 0.1mm. It is noted that these dimensions are exemplary only, and other dimensions are possible to provide for an implant device 100 that allows for placement into a brain cavity to be placed over a tumor-affected gyrus 63.

Generally, implant device 100 can be made in a variety of different dimensions to be pre-manufactured for different brain locations and different living beings, so that an appropriate device can be chosen for an affected brain gyrus 63. Also, it is possible to custom-made an implant device 100 for a specific living being, for example to custom manufacture guiding element 10 for specific dimensions of a target location of a target patient, for example by measuring the target location with a brain imaging technique, and thereafter machining or additively manufacturing the guiding element, for example by CNC machining or three-dimensionally (3D) printing a core of the guiding element 10 having suitable dimensions, and thereafter coating the manufactured core of the guiding element 10 with a layer or coating of biocompatible material, for example by different types of deposition techniques, and assembling the implant device 100 with the magnet structure 20 and lid 40. Before implantation, implant device 100 needs to be sterilized for long-term implantation.

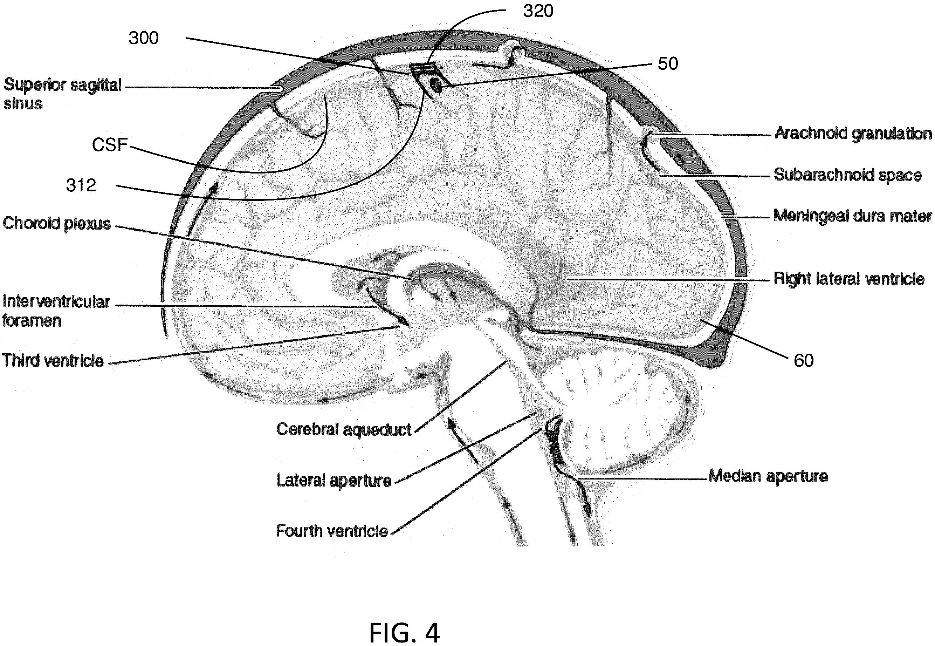

FIG. 2 shows an exemplary visualization of an implant device 100 according to another embodiment, placed at the implant site or location at a tumor 50, and serves also to further explain a principle of operation of implant device. Again for illustration purposes, a top portion of implant device is shown to have a relatively large height as compared to columns 12. In reality, a height of base plate 11, magnet structure 20, and lid 40 may have a height that is at least two (2) to eight (8) times smaller than a protruding height or depth of columns 12. FIG. 2 shows two sulci or fissures 64 of a brain 60 of a living being that are adjacent or neighboring a brain gyrus 64 that can be affected by a tumor 50. Columns 12 are configured to substantially protrude into sulci or fissures 64, for example to cover at least 60% of its depth. Moreover, lower surface 16 and legs of columns 12 have curved surface to at least somewhat match the curvature of affected brain gyrus 63. The skull and some of the layers of the brain 60 are not shown for visualization purposes, including pia matter, arachnoid, dura mater, and skull bone itself, but can be seen in FIG. 4.

Magnet structure 20 is configured to generate a permanent magnetic field that is exemplarily shown with field lines indicated by reference characters MF, and are principally arranged to be perpendicular to lower surface of magnet 22. With guiding element 10 having a ferromagnetic core 13, magnetic field MF is further directed to be perpendicular to a curved outer surface 18 of guiding element 10 to have different directions towards a center defined by brain gyrus 53, and thereby to be arranged to be somewhat perpendicular to an outer surface 67 of brain gyrus 63. Ferromagnetic core 13 therefore directs the magnetic field MF to be more concentrated towards a potential tumor 50 without the need of placing any ferromagnetic material or magnet into brain 60 itself, taking advantage of the naturally occurring environment of the brain with cavities in the form of sulci or fissures 64 around the brain gyrus 63. If no ferromagnetic core is present, guiding structure 10 still provides for fluidic guidance of particles 70 within collection area CA towards magnet structure 20, guiding particles 70 that move towards magnetic structure 20 up the fissures 64 to be captured by channels 14. This magnetic field MF allows to attract and move magnetic particles 70 located in the CSF by implant device 100, e.g. pathogenic micro- or nanoparticles that have made their way into the CSF, by the mechanical guidance given by guidance structure 10, and thereafter to be captured and held by channels 14, and possibly further directed to cavities 26, 27 inside structure 10. Magnetic field MF can progressively move such particles 70 away from brain gyrus 63 in the CSF towards channels 14 and outer surface 16 of guiding element 10, to bind, capture, and store particles 70 in channels 14, and also further move them into cavities of guiding element 10, such as passageways 26, 27 or collection chamber 326, to safely leave them there for a long duration, eliminating their inherent dangers to brain 60.

For example, particles 70 that can be captured at least partially with help of channels or cavities 14 for accumulating these particles 70 therein. Moreover, particles 70 can further move within CSF trough channels to move into passageways 27, 28 for additional accumulation space, and strong binding and attachment by arriving at outer surface of magnets 22, 24. Particle 70 can be different for example but not limited to graphite particles, mercury particles, iron particles, cobalt particles, tin particles, and uranium particles. The attraction and capturing of graphite particles may be of interest in light of the contamination of humans or other living beings by graphite bombings, and uranium particles are of interest in light of an exposure of a human to uranium radiation due to bombings or other nuclear fallout. Pope et al., "Health Effects of Particulate Air Pollution: Time for Reassessment?" Environmental Health Perspectives, Vol. 103, No. 5, 1995, pp. 472-480. Rev. Publ. Health Vol. 15, 1994, pp. 107-132. See also Hasegawa et al., "Health Effects of Radiation and Other Health Problems in the Aftermath of Nuclear Accidents, with an Emphasis on Fukushima," The Lancet, Vol. 386, No. 9992, 2015, pp. 479-488. Other particles that are not magnetic, for example lead particles or arsenic particles or particulate matter ("PM"), having strong pathogenic properties to different living beings, can be made magnetic by using an agent that can bind with these particles, the agent configured to be administered in vivo to a living being to reach the CSF, for example by using magnetic microparticles that are biosafe, to aid the magnetic capture process by implant device 100. Garraud et al., "Investigation of the Capture of Magnetic Particles from High-Viscosity Fluids Using Permanent Magnets," IEEE Transactions on Biomedical Engineering, Vol. 63, No. 2, 2015, pp. 372-378. Also, particles in air pollution, for example automotive exhaust pollution such as diesel exhaust particles and other particulate matter (PM) can also be potentially captured and isolated from the brain by implant device 100, as these can cause different types of neurodegenerative diseases, and prevent neuroinflammation. Block et al., "Air Pollution: Mechanisms of Neuroinflammation and CNS disease," Trends in Neurosciences, Vol. 32, No. 9, 2009, pp. 506-516.

Generally, implant device 100 serves as a magnetic collector and storage device for attracting, capturing, and making harmless several types of pathogenic, toxic, or otherwise damaging micro- and nanoparticles 70, down to the atomic level, by virtue of simple magnetic attraction of particles 70 located within the CSF and retention to magnet structure 20 via guiding structure 10. As the CSF surrounds the brain 60 and has an important protection function, presence of any such particles 70, also called particulate matter (PM) in the CSF has shown to have a high impact on the health of the living being. In addition, the presence of magnetic field MF around a brain tumor 50 can further help and assist in a strong reduction of pathogenic particles at the site or location of brain tumor 50. In this respect, implant device 100 serves a middle-term purpose to attract and collect damaging particles from the CSF, and a long-term purpose of radiating tumor 50 with a magnetic field to prevent and even extract particles from tumor 50 itself, again by means of CSF as a carrier of these particles 70.

FIG 3 shows an alternative embodiment of the implant device 200, where the guiding structure 210 includes side walls 230 for holding and accommodating the magnet structure 220 inside a magnet-holding space or cavity, that is located on upper surface 218. Moreover, a lid 240 is shown that has a smooth rounded surface. This embodiment allows to provide for a strong encapsulation, and at the same time eliminating any sharp edges or corners of the entire outer surface of implant device 200. Passageways 226, 227 are also formed within the magnet-holding space. A bonding agent or adhesive 232 is used to attach lid 240 to guiding structure 210, agent having biocompatible features. Moreover, columns 212 are shown to be substantially thinner that the columns 12 of the embodiment of FIG. 1.

FIG. 4 shows an exemplary cross-sectional view of a human brain 60 with another embodiment of implant device 300 implanted in the subarachnoid space SAS of the brain 60, such that implant device 100, 200 is surrounded by the CSF. As can be seen in this variant, implant device 300 is shaped to have columns 312 that protrude obliquely away from upper section, to accommodate the oblique nature of the arrangement of the brain fissures. Also, a height of the upper section is made such that the magnet structure 320 is fully accommodated inside the subarachnoid space SAS. Please note that this figure showing brain 60 has been made to illustrate accommodation and configuration of implant device 300 to different shapes of the brain 60 and the brain features, including gyrus 63 and fissures or suci 64, and is not used to visualize a real brain of a human being.

With respect to a method of operating the implant device 100, 200, 300, a first step of the method includes the choosing or the designing and manufacturing of an implant device 100, 200, 300 for a specific brain structure, tumor, and/or tumor location as discussed above. This step may require a substep in which the brain 60 of the tumor-affected person is imaged, for example by magnetic resonance imaging (MRI), to determine the geometry and dimensions of the site or location. A next step includes the sterilization of the implant device 100, 200, 300 for in vivo implantation via the skull and tissues. Next, a step can be performed in which a surgeon or other medical surgical practitioner or operator opens the intercranial space of patient, at the site or location for implantation, by opening scalp, skull, and the underlying brain tissues, including dura matter and the arachnoid, to access the desired subarachnoid space. In this step, different known methods can be used to access the intracranial space.

For example, surgical methods can be used that are analogous to the methods of implanting intracranial electrodes or subdural electrodes, for example as shown in Behrens et al., "Subdural and Depth Electrodes in the Presurgical Evaluation of Epilepsy," Acta Neurochirurgica, Vol. 128, No. 1-4, 1994, pp. 84-87, or see also "Advantages of Soft Subdural Implants for the Delivery of Electrochemical Neuromodulation Therapies to the Spinal Cord," Journal of Neural Engineering, Vol. 15, No. 2, 2018, p. 026024, these reference herewith incorporated by reference in its entirety.

Moreover, in a next step, the implant device 100, 200, 300 is placed into the subarachnoid space SAS with the legs or columns 12, 212, 312 to protrude into the fissures or sulci 63. This requires a gentle manual operation by surgeon, or can also be done by a robotic surgery assistance device, or a full robotic placement. Slight pressure needs to be applied to gently press lower surface 16 of guiding structure 10 to upper or outer surface 67 of affected brain gyrus 63. Thereafter, the surgical location is closed based on procedures that are known in the art. Surgical location in skull and tissues is healed, and implant device 100, 200, 300 starts to be operative, i.e. implant device 100, 200, 300 radiates the magnetic field to the CSF of the adjacent fissures 64, to adjacent areas of subarachnoid space SAS, and to small cavities and other channels in the brain mass 60 where the CSF is located. Implant device 100, 200, 300 can remain inside the body for a relatively long period of time, for example for a healing period of at least three (3) months or more, serving as a collector and storage unit for magnetic particles 70, and particles that have been made magnetic for this purpose.

Next, upon diagnosing or otherwise detecting an improved health status of patient, it is possible to remove implant device 100, 200, 300 permanently, by repeating the opening of implant location by the above-described surgical procedures. However, it is also possible that the implant device 100, 200, 300 remains in the living being. This step would allow to remove all particles 70 that have adhered to implant device 100, 200, 300 during the healing period. This step can be optional, and as long as there are no complications with implant device 100, 200, 300, or its operation can still be detected, for example by measuring the current strength of the magnetic flux density from outside the head of the patient, no removal may be necessary. After removal from living being, implant device 100, 200, 300 can be cleaned from particulate matter, can be sterilized, and reused for the same or other living being.

As compared to background art particle cleaning techniques of the CSF, and tumor removal techniques, and also methods of preventing tumors, the present device, system, and method presents several important advantages. First, while the method, device, or system does require an *in vivo* operation for its placement to be in close proximity of the brain inside the CSF, the method is only partially invasive to the human body, as no chemicals or pharmaceuticals are used, and therefore any inherent side effects related thereto are reduced or entirely eliminated. Also, no mechanical changes to the human body structure are needed, other than the surgery for its placement, such as mechanical removal of tumors, or radiation techniques. The present device, system, and method also only generates a relatively weak magnetic field that is not harmful for its operating environment, i.e. in proximity of the brain. It serves as a healing implant device that acts over a relatively long period of time to remove and collect pathogens in the form of magnetic particles, or particles that are made magnetic for the collection purpose, with no impact on the brain activity. By virtue of the magnetic action on particles in the CSF, and also particles that are located inside brain fissures within the CSF, the device, system and method can slowly collect such particles with no mechanical action needed.

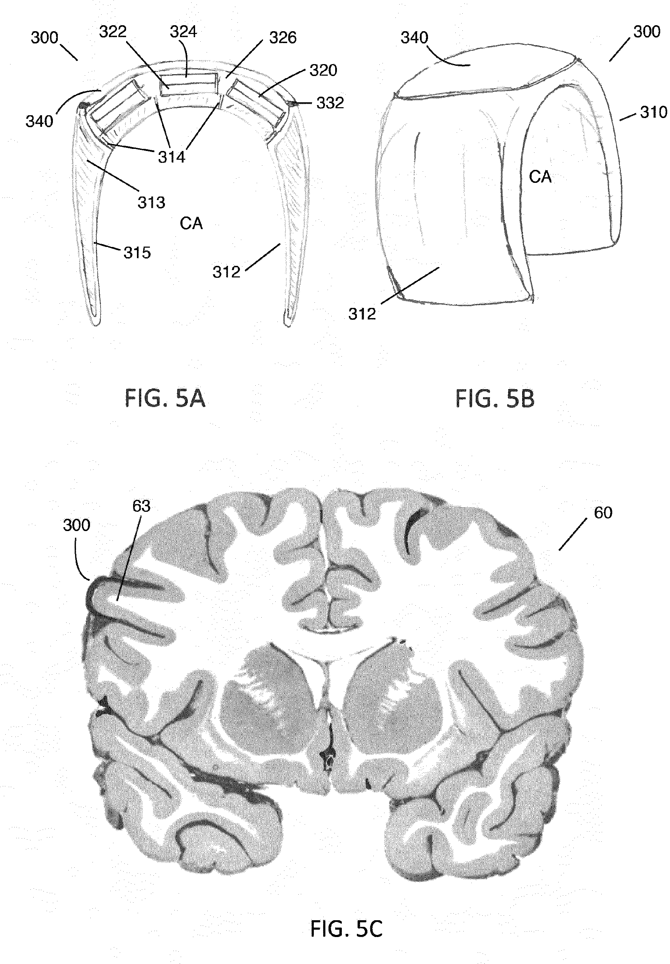

FIGs. 5A-5C show different views of an another embodiment of implant device 300, where the legs 312 of guiding structure 310 are formed to be longitudinal and bent or curved tabs or ledges, to form a U-shaped structure. In the cross-sectional view of FIG. 5A, three stacks of magnets as the magnet structure 320 are shown, each with a top magnet 324 and a bottom magnet 322. More than two stacked magnets are also possible. Exemplary, there could be two or three rows of magnet stacks, to form a total of six (6) or nine (9) tablet stacks. Different channels 314 are shown that lead to opening or cavity 326 in the upper section of guiding structure 310. Also, inside tabs 312 and a section of body portion of guiding structure 310 between magnet structure 320 and accommodating space for brain gyrus 63, a core 313 is arranged that is coated by a biocompatible coating 315. Preferably, the core has ferromagnetic properties. In this variant, each stack of magnets can be arranged at a different angle to each other, in a way that a main axis of the magnetic field MF per stack is directed towards a center point that would be inside a brain gyrus 63. This allows to further concentrate the magnetic field MF to a specific location, for example a location of a tumor 50. Also, this arranged allows to provide for a design of implant device 300 that has more rounded structures, avoiding any sharp or other rugged edges, as shown in FIG. 5B. In this respect, upper surface formed by lid 340 can be curved, cylindrical, or spherical. A bonding or adhesive material 332 can be used to attach lid 340 to guiding structure 310, but lid 340 can also be welded or fixedly attached to guiding structure 310. Magnets of magnet structure 220 can be attached to lid 340, or can be attached to each other by an adhesive, without touching the lid 340.

FIG. 6 shows a cerebro-spinal brain fluid treatment system 400 for attracting, collecting, and holding particles that are located in the CSF, the system 400 including a magnet device 420 to be placed in close proximity of a skull SK of a living being, for *ex corporis* application of magnet device 420, an implantable guiding structure 410, in this variant with no active magnets, made of a biocompatible material or coated with a biocompatible material. The implantable guiding structure 410 includes a base portion 411, one or more protruding body elements 412, and a plurality of channels 414, each one of the plurality of channels having an opening orifice at an outer surface of the implantable guiding structure 410 that is configured to face the cerebrum. Channels 414 are arranged such that their exit orifices lead to a collection chamber 426 inside the base portion 411 of implantable guiding structure 410. Magnet device 420 can be made of permanent magnets, or active electromagnets, as shown.

Particles 70 can be attracted by magnet device 420, and the ones located inside the collection area CA of guiding structure 410 are guided and directed to channels 414, can enter the channels 414 to be collected in collection chamber 426. In the case active electromagnets are used, a power device 470 is necessary that is in operative connection with magnet device 420, for example by being arranged directly at magnet device 420 or remotely and cabled to magnet device, for example including a battery or other type of power supply, and a power converter to apply the requisite power to magnet device 420. As compared to the solutions described above where the magnets are *in vivo* and in contact with guiding structure 10, 210, 310, this variant requires a much stronger magnetic field MF to be generated by device 420 for proper operation, but presents the advantage of providing less material, less weight, and less volume to the implant site. Another advantage is the possibility of removal of magnet device 420 after a healing period, without the need of surgical operation. For this purpose, collection chamber 426 or channels 414 can be equipped with a membrane, valve, or filter for letting particles 70 pass to move closer towards a location of magnet device 420, but prevents them from leaving collection chamber or channels 414, for example a cell membrane configured as a filter for the specific particulate matter that needs to be captured.

FIG. 7 shows an exemplary cross-sectional view of another embodiment of implant device, with only one magnet 520 arranged in a lid 540, lid 540 configured to be screwably attached to guiding structure 510 by a threading 540. Also, this embodiment includes a collection chamber 526 that is arranged between lower surface of magnet 520 and upper surface of base portion of guiding structure 510, providing for additional collection space. Entrance orifice of channel 514 can be tapered or have other type of funneling function, and can also be equipped with a permeable membrane for different types of particles to serve as a valve, for letting particles 70 into collection chamber 526. Instead of having a single channel, it is also possible to have a plurality of channels, or to have a porous body portion between collection chamber 526 and collection area CA.

While the invention has been disclosed with reference to certain preferred embodiments, numerous modifications, alterations, and changes to the described embodiments, and equivalents thereof, are possible without departing from the sphere and scope of the invention. Accordingly, it is intended that the invention not be limited to the described embodiments, and be given the broadest reasonable interpretation in accordance with the language of the appended claims.

## Claims

1. An implant device for placement into a brain cavity of a living being between the meninges and the cerebrum to be in contact with a cerebro-spinal fluid, the implant device comprising:
a magnet structure having a biocompatible coating; and
a guiding structure made of a biocompatible material or coated with a biocompatible material, an outer surface of the guiding structure configured to be in contact with the cerebro-spinal fluid and forming a collection area,
wherein the guiding structure includes at least one channel having an opening at an outer surface of the guiding structure that is configured to face the collection area.

2. The implant device of claim 1, wherein the guiding structure includes a center body portion facing the magnet structure, and a protruding body element.

3. The implant device of claim 2, wherein the protruding body element includes two protruding columns or tabs, a distance between the two protruding columns or tabs dimensioned such that each one of the two protruding columns or tabs is configured to protrude into a corresponding brain sulcus that is adjacent to a brain gyrus.

4. The implant device of claim 2, wherein the protruding body element includes four protruding columns with two column pairs, a distance between two protruding columns that form the column pairs dimensioned such that each one of the two protruding columns is configured to protrude into a corresponding brain sulcus that is adjacent to a brain gyrus.

5. The implant device of claim 1, wherein the magnet structure includes a permanent magnet, and the guiding structure includes a collection cavity and/or a passageway that is in fluidic contact with the channel, and in contact with an outer surface of the magnet structure.

6. The implant device of claim 1, wherein the magnet structure includes a plurality of magnets arranged adjacent to each other, the magnets arranged such that there is at least one cavity between side walls of two neighboring magnets.

7. The implant device of claim 6, wherein the plurality of magnets are arranged as a first stack of magnets, each one of the magnets from the first stack being in contact with the guiding structure, the magnet structure further including a second stack of magnets in contact with the first set of magnets opposite of the side that contacts the guiding structure.

8. The implant device of claim 6, wherein the channel forms a fluidic connection between the brain cavity and the outer surface of the plurality of magnets.

9. The implant device of claim 1, wherein the guiding structure includes a ferromagnetic core.

10. The implant device of claim 1, wherein the channel includes a membrane for passage of particulate matter.

11. An cerebro-spinal brain fluid treatment system comprising:
a magnet device to be placed in close proximity of a skull of a living being; and
an implantable guiding structure made of a biocompatible material or coated with a biocompatible material, configured to be in contact with the cerebro-spinal fluid and forming a collection area,
the implantable guiding structure including,
a base portion,
a protruding body element, and
at least one channel having an opening at an outer surface of the implantable guiding structure that is configured to face the collection area.

12. The system of claim 11, wherein the magnet device includes at least one of a permanent magnet and/or an electromagnet.

13. The system of claim 11, wherein the implantable guiding structure includes a ferromagnetic core.

14. The system of claim 11, wherein the implantable guiding structure includes a collection cavity, wherein the channel provide for a fluidic connection between the brain cavity and the collection cavity.

15. The system of claim 11, wherein the channels include a membrane for passage of particulate matter.
